(19) **Europäisches Patentamt / European Patent Office / Office européen des brevets**

(11) **EP 2 197 334 B1**

(12) **EUROPEAN PATENT SPECIFICATION**

(45) Date of publication and mention
of the grant of the patent:
**05.07.2017  Bulletin 2017/27**

(51) Int Cl.:
*A61B 1/04* *(2006.01)*      *A61B 3/14* *(2006.01)*
*A61B 1/227* *(2006.01)*    *A61B 1/00* *(2006.01)*

(21) Application number: **08775522.9**

(22) Date of filing: **30.06.2008**

(86) International application number:
**PCT/FI2008/050397**

(87) International publication number:
**WO 2009/004115 (08.01.2009 Gazette 2009/02)**

(54) **PRODUCING AN IMAGE**

ERZEUGUNG EINES BILDS

PRODUCTION D'IMAGE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **29.06.2007  FI 20075499**

(43) Date of publication of application:
**23.06.2010  Bulletin 2010/25**

(73) Proprietor: **Optomed Oy
90230 Oulu (FI)**

(72) Inventor: **POHJANEN, Petri
90570 Oulu (FI)**

(74) Representative: **Kolster Oy Ab
Salmisaarenaukio 1
00180 Helsinki (FI)**

(56) References cited:
**EP-A1- 1 347 638        WO-A1-99/42030
DE-A1- 19 723 442        JP-A- 02 020 817
US-A- 5 860 912        US-A- 5 871 439
US-A- 6 092 722        US-A1- 2004 030 221
US-A1- 2007 030 345**

## Description

FIELD

The invention relates to producing an image of an organ by using a camera unit.

BACKGROUND

Digital optical instruments may be used as assistance in examining organs, such as the eye, the ear and the skin. For example, a digital image in an electronic format may be produced of an object imaged with a portable camera unit suitable for ophtalmoscopy, otoscopy or dermatoscopy. However, the functions of known imaging devices are manual and intended for one kind of use. This being the case, a device intended for imaging the eye, for example, is not intended for imaging the skin or the ear. Patent document US 20070030345 presents a universal scope reader. Patent document US 6092722 presents a method and a device for the automatic indentification of components of medical apparatus system. Further imaging instruments are disclosed in document WO99/42030.

Problems are associated with such solutions. Although the imaging device is able to produce a fixed image or video image of an organ for the imaging of which the imaging device is not intended, successful imaging is difficult and the quality of the image is not good.

BRIEF DESCRIPTION

The invention is defined in the claims. Other embodiments are merely exemplary.

The method and system of the disclosure provide a plurality of advantages. Since the imaging optics can be adapted for each imaging, imaging is easy and image quality good. The camera unit or another part of the system is able to automatically affect image production, whereby the imaging conditions can be improved and/or image processing enhanced for obtaining a successful image.

LIST OF FIGURES

In the following, the invention will be described in more detail in connection with preferred embodiments with reference to the accompanying drawings, in which

Figure 1 shows an eye examination performed with a camera unit,
Figure 2A shows the imaging of an eye or the like,
Figure 2B shows the imaging of an ear or the like,
Figure 3 shows the imaging of a nose or the like,
Figure 4 shows a block diagram of a camera unit,
Figure 5A shows a camera unit and a docking station,
Figure 5B shows a docking station,
Figure 6 shows data transfer between a camera unit and a server, with the coserver outside the institution,
Figure 7 shows data transfer between a camera unit and a server, with the coserver inside the institution, and
Figure 8 shows a flow diagram of the method.

DESCRIPTION OF EMBODIMENTS

The camera unit of the analytical device may be mainly similar to the solutions disclosed in Finnish published patents FI 107120 and FI 2002 12233, for which reason all properties of a camera unit, known *per se,* are not described in more detail in the present disclosure; instead, the focus is on the characteristics of the solution presented that differ from what is known.

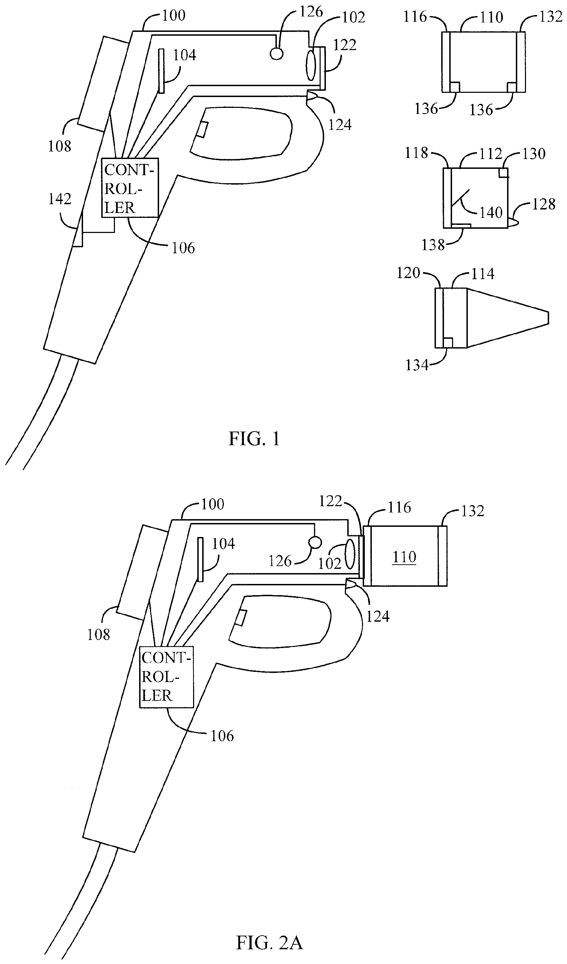

Let us first study the solution disclosed by means of Figure 1. In this example, the analytical device is represented by a camera unit 100, which may be a portable camera based on digital technology. The camera unit 100 of the analytical device may comprise an objective 102 for producing an image of an organ to a detector 104 of the camera unit 100. With the analytical device in working order, the detector 104 is able to produce an image in an electronic format of the organ. The image produced by the detector 104 may be input in a controller 106 of the camera unit 100, which controller may comprise a processor and memory and is intended to control the camera unit 100, process and store the image and any other data. From the controller 106, the image may be input in a display 108 of the camera unit 100 for displaying the image and any other data. The detector 104 of the camera unit 100 may be a CCD (Charge Coupled Device) sensor or a CMOS (Complementary Metal Oxide Semiconductor) sensor, and the camera unit 100 is able to produce fixed images or video image.

The analytical device comprises not only the camera unit 100, but also at least one optical component 110 to 114, which is connectable to the camera unit 100. Each optical component 110 to 114 is intended, alone or together with one of the other optical components 110 to 114, for imaging a predetermined organ. In accordance with the object to be analyzed, the camera unit 100 may be provided with a suitable optical component. Fastened to the camera unit 100, each of these optical components 110 to 114 is able to communicate with the camera unit 100 and/or with each other. Furthermore, each optical component 110 to 114 may communicate with peripheral devices. Each optical component 110 to 114 is able, alone or together with one or more other optical components 110 to 114, to control image production, processing and storage.

Each optical component 110 to 114 includes a data structure 116 to 120, which includes data associated with the optical component 110 to 114. The data structure 116 to 120 may be disposed in a frame structure of the optical component 110 to 114 or in at least one actual component used in image production, such as a lens.

The optical component 110 to 114 may comprise for instance one or more image-producing elements, such as a lens or a mirror, and the optical component 110 to 114 may serve as an additional objective of the camera unit 100.

The data structure 116 to 120 may be an electromechanical structure, for example, which mechanically fastens the optical component 110 to 114 to the camera unit 100 and establishes an electric connection between the camera unit 100 and the optical component 110 to 114. Connecting the data structure 116 to 120 against a counterpart 122 of the camera unit 100 enables transfer of the data associated with the optical component 110 to 114 from the data structure 116 to 120 via the counterpart 122, for instance along a conductor, to the controller 106. In this case, the data structure 116 to 120 and the counterpart 122 of the camera unit 100 may comprise one or more electric contact surfaces, for example. The electric connection may be specific to each optical component 110 to 114 or component type. Via the contact surfaces, the camera unit 100 is able to connect electricity to the data structure 116 to 120, and the response of the data structure 116 to 120 to an electric signal from the camera unit 100 indicates data specific to each optical component 110 to 114.

A different optical component 110 to 114 may be arranged for the imaging of different organs, whereby each optical component 110 to 114 has a different connection. The connections may differ from each other as regards resistance, capacitance and inductance, for example, which affects the current or voltage detected by the camera unit 100, for example. Instead of such an analog coding, digital coding may also be used for distinguishing the optical components 110 to 114 from each other.

The data structure 116 to 120 may also be a memory circuit, for example, which comprises data specific to each optical component 110 to 114. The data structure 116 to 120 may be a USB memory, for example, and the camera unit 100 may comprise a connector for the USB memory as the counterpart 122. The data associated with the optical component 110 to 114 may be transferred from the memory circuit to the controller 106 of the camera unit 100, whereby the controller is able to control the camera unit 100 based on the data.

Reading the data included in the data structure 116 to 120 does not necessarily require a galvanic contact between the camera unit 100 and the data structure 116 to 120. In this case, the data associated with the optical component 110 to 114 may be read from the data structure 116 to 120 capacitively, inductively or optically, for example. The data structure 116 to 120 may be a bar code read by a bar code reader in the camera unit 100. The bar code may also be read from an image produced by means of an image processing program comprised by the camera unit 100. The bar code may be detectable at a different wavelength than the one used for imaging the organ. The bar code may be identified by means of infrared radiation, for example, when the organ is imaged with visible light. In this manner, the bar code does not interfere with the imaging of the organ.

The data structure 116 to 120 may also be an optically detectable characteristic of each optical component 110 to 114, such as some distortion of the image, including spherical aberration, astigmatism, coma, curvature of image field, distortion (pin cushion and barrel distortion), chromatic aberration, and/or an aberration of some higher degree (terms exceeding the third degree of Snell's law), for example. In addition, the data structure 116 to 120 may be a structural distortion in the lens. Structural distortions in a lens include geometric distortions (projections or holes), lines, impurities and bubbles. Each of these distortions may affect the image produced in an identifiable manner. Once the camera unit 100 has identified a distortion specific to a given optical component 110 to 114, the optical component 110 to 114 can be identified, the identifier data stored, and/or the data be utilized in image processing.

The memory circuit may also be an RFID identifier (Radio Frequency Identification), which may also be called an RF tag. A passive RFID identifier has no special power source; instead, its operation depends on energy from a reader device, i.e. in this case the camera unit 100. Energy may be supplied to the RFID identifier through a conductor from e.g. a battery or, in a wireless solution, the energy of an inquiry signal of the identifier data may be utilized, for example.

The camera unit 100 is able to compare the image produced with a given optical component 110 to 114 with a reference image, which may be stored in the memory of the camera unit 100. The comparison could be associated with a distortion, the contrast or the brightness, for example, in the different parts of the image. In this manner, information on the optical characteristics of each optical component 110 to 114, such as the refraction indices of the lenses, can be obtained. Faults present in the image may also be corrected.

Thus, the data structure 116 to 120 of each optical component 110 to 114 is able to send data associated with the optical component 110 to 114 to the camera unit 100 when at least one optical component 110 to 114 is connected to the camera unit 100. By means of data associated with each connected optical component 110 to 114, the data structure 116 to 120 is able to directly or indirectly (by means of the controller 106, the controller 532 or the server 602, for example) control the image production of an organ performed with the camera unit 100.

One or more optical components 110 to 114 may also comprise a detecting sensor 138, to which the optical radiation may be guided either directly or by means of a mirror 140, for example. The mirror 140 may be partly permeable. The detecting sensor 138 may also be so small that it covers only part of the optical radiation passing through the optical component 110 to 114, in which case the optical radiation also reaches the detector 104. In one optical component 110 to 114, there may be more than one detecting sensor and they may be in a wired connection, for example, to the controller 106 when the optical component 110 to 114 is connected to the camera unit 100. When fastened to the camera unit 100, the detecting sensor 138 may be activated to operate with electrical energy from the camera unit 100 and it can be used to produce an image of the organ to be imaged. The detecting sensor 138 may operate at a different wavelength than the detector 104. This being so, the detecting sensor 138 may produce an image in infrared light, for example, and the detector 104 in visible light, for example. The mirror 140 may reflect infrared radiation extremely well and at the same time let a significantly large part of visible light through. The image data of the detecting sensor 138 and the image data of the detector 104 may be processed and/or combined and utilized together or separately. The detecting sensor 138 may be a CCD or a CMOS element, for example.

In the case of Figure 1, where no optical components 110 to 114 are fastened to the camera unit 100, the camera unit 100 may be used for imaging the skin.

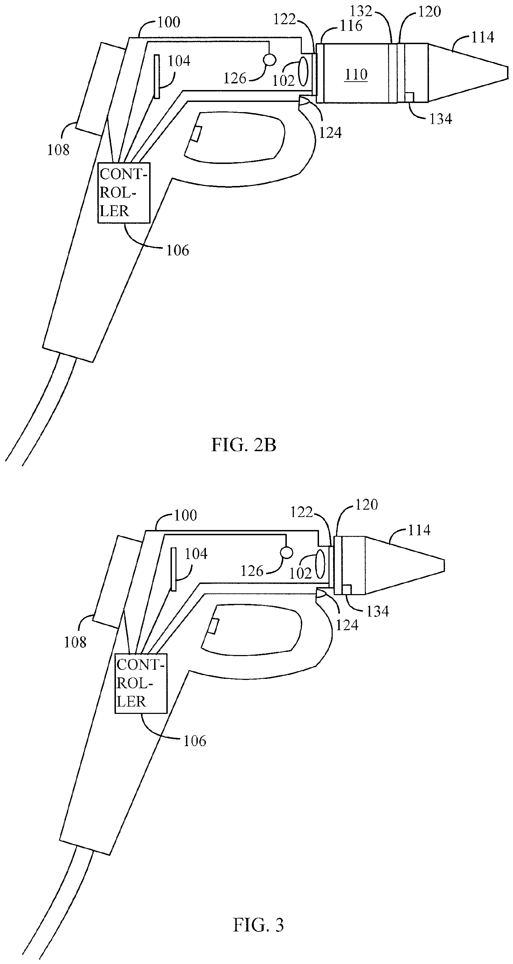

Figures 2A, 2B and 3 show the imaging of different organs. In Figure 2A, one may imagine that an eye is being imaged, and that an optical component 110, suitable for the imaging of the fundus of the eye, is fastened to the camera unit 100. In this case, the data structure 116 of the optical component 110 suitable for imaging the eye is able, by means of a mechanical connection specific to this component and representing data associated with said optical component and together with a counterpart 122, to switch on one or more optical radiation sources 124 at the front of the camera unit 100, in order for it to illuminate the eye. Alternatively, the radiation source 124 may be switched on such that the data associated with the optical component 110 of the data structure 116 is transmitted along a conductor or wirelessly to the counterpart 122 of the camera unit 100 and from there to the controller 106 or directly to the controller 106, which switches the radiation source 124 on based on the data associated with the optical component 110. The radiation source 124 may be switched on automatically. In this case, the optical radiation source 126 within the camera unit 100 may be correspondingly switched on or off. The radiation sources 124, 126 may be radiators of the range of visible light or of the range of infrared radiation, for example.

Figure 2B shows an implementation, wherein two optical components 110, 112 are fastened together and the combination fastened to the camera unit 100. The optical components 110, 112 are in contact with each other by means of the counterpart 132 of the optical component 110 and the data structure 120. In this case, one may imagine that when an optical component 114, suitable for imaging the ear, is fastened to an optical component 110 suitable for imaging the fundus of the eye, an efficient device for imaging the tympanic membrane and the external acoustic meatus can be generated. In such a situation, the optical radiation of the radiation source 124 at the front of the camera unit 100 cannot enter the external acoustic meatus (the nose). In this case, by means of the data associated with the optical components 110, 114, the data structures 116, 120 of the optical components 110, 114 are able to switch the radiation source 124 off and the radiation source 126 within the camera unit 100 on, since the radiation of the radiation source 126 is able to propagate through the nozzle piece into the ear (the nose). The camera unit 100, a computer 410 or a server 602 is able to utilize the data of a plurality of data structures 116, 120 for processing image data. Instead of switching on or off, the brightness, the direction or the tone of the illumination can also be adjusted, if the optical radiation of the radiation source 124 can be directed to the external acoustic meatus (the nose). Furthermore, if the optical characteristics, such as the focal distance, the polarization and the optical transmission band, for example, are known, the illumination characteristics can be affected versatilely.

Figure 3 shows an imaging event, which also shows the imaging of a cavity of the body. The optical component 114 alone may correspond to a nozzle piece used in the examination of the nose (the ear or the mouth), for example, and whose tip portion is inserted into the nasal cavity. In such a situation, the optical radiation of the radiation source 124 at the front of the camera unit 100 cannot enter the nasal cavity. In this case, the data structure 120 of the optical component 110 is able, by means of the data associated with the optical component 114, to switch off the radiation source 124 and the radiation source 126 within the camera unit 100 on, since the radiation of the radiation source 126 cannot propagate through the nozzle piece into the nose. Instead of switching on or off, the brightness, the direction or the tone of the illumination can also be adjusted. Furthermore, if the optical characteristics, such as the focal distance, the polarization and the optical transmission band, for example, are known, the illumination characteristics can be affected versatilely.

The data structure 120 of the optical component 114 suitable for imaging the nose or the ear may directly, by means of an electrical and mechanical connection specific to this component and representing data associated with said optical component and together with a counterpart 122, switch off one or more optical radiation source 124 at the front of the camera unit 100. Alternatively, the radiation source 124 may be switched off such that the data associated with the optical component 114 of the data structure 120 is transmitted by wire or wirelessly to the controller 106 of the camera unit 100, which switches off the radiation source 124 based on the data associated with the optical component 114. In this case, the radiation source 126 within the camera unit 100 may be switched on in a corresponding manner. Instead of switching on or off, the brightness, the direction and the tone of the illumination can also be adjusted. Furthermore, if the optical characteristics, such as the focal distance, the polarization and the optical transmission band, for example, are known, the illumination characteristics can be affected versatilely.

When the skin is imaged, no separate optical component 110 to 114 needs be fastened to the camera unit 100, but the skin may be imaged also directly with an objective 102 fixedly comprised by the camera unit 100. On the other hand, a suitable optical component 110 to 114 may be used for imaging the skin and switch on or off a suitable illumination by means of the data associated with the optical component and comprised by the data structure 116 to 120.

In stead of or in addition to illumination, the data associated with an optical component of the data structure 116 to 120 may be utilized by the controller 106 of the camera unit, which controller may also serve as an image processing unit, for processing an image taken with the detector 104 of an organ. In the image, the brightness, the exposure, the contrast, the colour shades or the colouring of the image may be modified, for example. Instead or in addition, the zooming may also be modified.

Let us now study the control of image producing more generally. The data associated with the optical components 110 to 114 intended for different uses enables many aspects of image producing when at least one of them is fastened to the camera unit 100.

In an implementation, each optical component 110 to 114 comprises identification data enabling the automatic identification of each optical component 110 to 114. The identifier may determine the individual optical components or the optical components may be identified by types according to the intended procedure or purpose. In this case, for example, an optical component intended for eye examination enables automatic 'ocular optics' or 'image of eye' data to be associated with the image.

Based on the data associated with the optical components 110 to 114, an image may be processed versatilely.

In an implementation, the camera unit 100 is able to modify the brightness of the image. In this case, when the image is being displayed, the intensity of each pixel on the display is increased or reduced.

In an implementation, the camera unit 100 is able to modify the contrast of the image. In this case, when the image is being displayed, the intensity differences of each pixel on the display are increased or reduced. For example, if an optical component 110 to 114 causes the edge areas of an image produced to be darker than the mid area of the image, data about such a characteristic may be stored in said optical component 110 to 114. When said optical component 110 to 114 is used, the data of the characteristic associated with said optical component 110 to 114 may be transferred to the camera unit 100, the computer 410 or the server 602, for example, wherein the contrast of the image can be modified based on the data. In this case, for example, the contrast can be reduced in the edge areas. Similarly, the brightness may also be adjusted in various manners in the different parts of the image. A plurality of images taken of the same object may also be stacked or an HDR image (High Dynamic Range Image) may be produced. Similarly, a composite can be produced also from video image.

In an implementation, the camera unit 100 is able to produce an image with predetermined exposure values. The exposure values may be measured by means of the detector 106 of the camera unit 100, for example, but the camera unit 100 may also comprise one or more separate photometers. Measurement of exposure may be used to measure the illumination of the object on the band of visible light and/or on the band of infrared radiation of optical radiation.

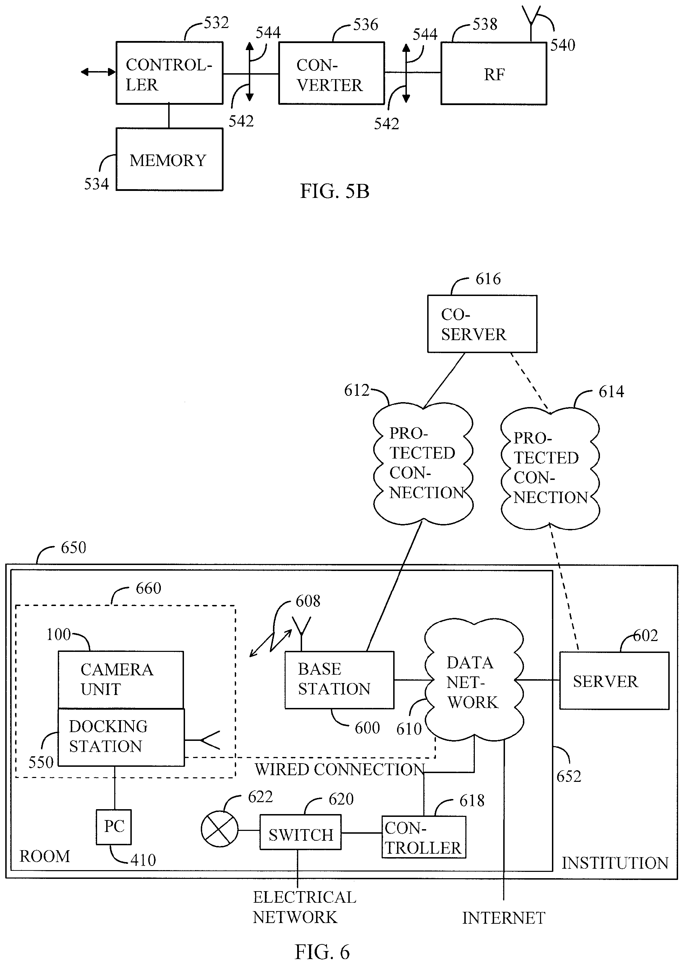

In an implementation, the data associated with an optical component 110 to 114 fastened to the camera unit 100 may be used to control the illumination of the environment and thus also affect the illumination of the organ to be imaged. In this case, the data on the optical component 110 to 114 is transferred to a controller 618 controlling the lights of an examination room 652, for example, said controller controlling a switch 620 on or off. When the switch 620 is controlled off, electric power is connected to a light source 622 in the examination room, and the light is on. When the switch 620 is controlled on, electric power does not enter the light source 622, and the light is off (Figure 6). The electric power may originate from an electrical network, for example. The controller 618 may also otherwise adjust the illumination in the examination room 652. In this case, the illumination may be increased or reduced, or the colour or colour shade of the illumination may be adjusted. When the camera unit 100 and the object to be imaged are in the vicinity of the light source 622 of the examination room, the light source 622 may be dimmed, for example. Similarly, if the camera unit 100 is far away from the light source 622, the light source 622 may be controlled to illuminate more strongly, for example.

In an implementation, only part of the detection area of the detector 104 is selected to the image and the rest is cropped from the image to be produced by means of the data associated with the optical component of the data structure 116 to 120. In this case, the image may comprise the mid part of the detection area of the detector, for example, i.e. the image may be thought to be zoomed. Accordingly, if the pixels of the entire detection area of the detector 104 form a matrix n x m, pixels k x p of the matrix may be selected to the image, wherein either $k \leq n$ is true for k or $p \leq m$ is true for p or $k \leq n$, $p \leq m$ is true for both k and p. Thus, if the detector 104 comprises a pixel matrix having a size of 1000 x 1200 pixels, the image may be produced based on the values of 500 x 600 pixels, for example.

In an implementation, the image produced is coloured in the desired manner, either entirely or partly. The colouring, as well as at least part of the other procedures associated with image producing, may be performed in the camera unit 100, in a separate computer 410, in a docking station 550, in a base station 600 or in the server 602. The colouring may be such that, when the eye is being imaged, the image is coloured orange, when the ear is being imaged, it is coloured red, and when the skin is being imaged, it is coloured blue, for example.

In an implementation, the data associated with the optical component 110 to 114 may be used to determine information about the object to be imaged, such as the nose, the mouth, the ear, the skin etc., since each optical component 110 to 114, alone or together with one or more other predetermined optical components 110 to 114, may be intended to image a predetermined organ. This being so, the optical component intended to examine the eye, for example, enables automatic 'ocular optics' or 'image of eye' data to be associated with the image. When the camera unit 100 is aware of the object to be imaged by means of the data associated with one or more optical components 110 to 114, the camera unit 100 is able to use image processing operations to automatically identify and optionally mark predetermined shapes in the object to be imaged with colour, for example. When the image is displayed, the points marked are clearly distinguishable, and may be the characteristics of a disease, for example.

The success of the diagnosis can be monitored by means of data received from one or more optical components. If the patient's symptoms are in the eye, but the ear was imaged with the camera unit 100, the conclusion is that the procedure was not right. It is also feasible that the server 602 has transmitted information about the patient and his/her ailment to the camera unit 100 in the DICOM format, for example. In this case, the camera unit 100 controls the imaging to only what the patient has complained of, i.e. in this case, the eye. If another optical component 110 to 114 than an optical component suitable for imaging the object according to the ailment (the eye) is fastened to the camera unit 100, the camera unit 100 warns the imager with an acoustic signal and/or a warning message on the display of the camera unit 100.

A patient data system of a hospital, for example, collecting the image by means of the data received from one or more optical components, is able to generate both statistics and invoicing data.

In an implementation, the persons authorized to use the image produced can be determined by means of the data associated with one or more optical components 110 to 114 fastened to the camera unit 100, and prevent other people from using the image. This being so, the image may be protected for instance with a password, which is known only to predetermined people. In this case, the image may be automatically directed to a predetermined one or more physicians. The physician or a group of physicians, to whom the image is directed, may be for instance an individual physician desired by the patient, the physician taking the image or physicians specialized in the organ being imaged. In this case, the specialist physicians are not necessarily determined according to the name of the physicians, but according to the professional specialization.

In image production, the imaging performance can be affected versatilely based on the data associated with the optical components 110 to 114.

In an implementation, the reliability of the end user concerning the use of the camera unit 100 can be analyzed. The data associated with each optical component 110 to 114 implies the organ to be imaged or at least the kind of imaging event involved. In this case, the imaging should be performed in a manner according to the imaging object or the imaging event. In an implementation, the camera unit 100 detects 'the wrong type of' movements in relation to the organ to be imaged or the imaging event, for example. Detection of movement can be performed from a video image produced by the camera unit 100 with the controller 106, for example. The movements measured may be compared with a reference based on the data associated with the optical component 110 to 114. If, for example, the camera unit 100 is moved more than a predetermined highest reference value, the movement may be interpreted to mean that the user should be instructed on the use of the camera unit 100. In this case, the camera unit 100 may display, for instance on the display 108 thereof, instructions on the use of the camera unit 100 or present a request to receive instructions on the use of the camera unit 100. Generally, the user is presented a message about the uncertainty of the use if the measurement result of the movements of the camera unit 100 is higher than the highest reference value based on the data associated with one or more optical components 110 to 114. The measurement result of the movements of the camera unit 100 may represent the extent, the velocity, the acceleration, etc. of the movements. The measurement results may be determined by measuring video image.

Alternatively or in addition, one or more optical components 110 to 114 comprise an acceleration sensor unit for measuring the movement of the camera unit 100. In addition to acceleration, the acceleration sensor is able to measure the velocity v of the movement by integrating acceleration a over time $t_0$ to $t_1$, $v = \int_{t_0}^{t_1} a\,dt$ .

In image production, the object to be imaged can be affected versatilely based on the data associated with the optical components 110 to 114. In an implementation, the camera unit 100 is able to ensure that the device is not too close to the object being imaged or far from the object being imaged. This may be implemented for instance by means of focusing, since each optical component 110 to 114 affects focusing. For example, when the camera unit 100 has received identification data about which optical component(s) 110 to 114 is/are as arranged as the objective, the camera unit 100 is able to determine a suitable focusing distance between the object to be measured and the camera unit 100, for example. When the camera unit 100 is used to image the desired object, it can be detected whether the device is already too close to the object or that it approaches the object too rapidly, whereby automatic focusing cannot catch up. Alternatively or in addition, each optical component 110 to 114 may also comprise a distance meter 128 for generating distance data. The distance meter 128 may be in a wired connection to the controller 106 when the optical component is connected to the camera unit 100. Distance data may be transferred to the controller 106 of the camera unit 100, which controller may utilize the distance data in controlling the camera unit.

The camera unit 100 is able to perform anticipation of the focusing of the object, for example, by means of the focusing distance data or the distance measurement data. If the focusing or the zooming motors and the focusing control are located in each optical component 110 to 114, the optical component 110 to 114 or the controller 106 of the camera unit 100 is able to predetermine, based on the movement, when the object to be imaged is in focus and thus enable image production although the object is not yet in a sharp area. This also allows the image function to be kept activated (the imaging trigger can be kept at the bottom) the entire time, but only when the object is in focus does the camera unit 100 perform the imaging. It is also feasible to take images the entire time when the imaging function is active, but only the images determined sharp by the camera unit 100 are marked successful. Successful images may be stored.

In image producing, the rest of the functionality can be affected versatilely based on the data associated with the optical components 110 to 114. In an implementation, the data measured by one or more optical components 110 to 114 can be transferred to the camera unit 100 in connection with image production. In this case, the optical component 110 to 114 may comprise a thermometer 130, for example, for measuring the temperature of the environment or the object to be measured. The thermometer 130 may be in a wired connection to the controller 106 when the optical component is connected to the camera unit 100. The measurement may be performed with an IR thermometer (InfraRed), for example. The temperature measured can be attached to the image data as numerical data, for example. Image data may also be processed as a function of the temperature in the desired manner. In this case, the entire image or part of the image may be shaded according to the temperature and thus the temperature can be concluded when studying the image later.

In an implementation, the camera unit 100 is able to ensure that all shields required in connection with the imaging, such as funnels required in ear examination, are in position or replaced after the previous imaging. If a funnel required in ear examination, for example, is not replaced when the patient changes, the camera unit 100 may warn the physician with a signal and/or a warning on the display.

The user of the camera unit 100 may also control the functions of the camera unit 100 relative to the imaging requirements associated with the imaging object from a menu of the camera unit 100. In this case, the user may input for instance the data on the imaging object, the amount of light required in the imaging object, the requirement of colour change to be used in the imaging object, and the like. The graphical interface of the camera unit 100 may also change according to the optical component 110 to 114, controlled by the optical component 110 to 114. In this manner, the data to be input by means of a menu may be different when different optical components 110 to 114 are used.

In an implementation, the position of use of the camera unit 100 can be identified. Since different optical components 110 to 114 receive light with different numerical openings and produce a different image, it is possible to determine the direction of the light received and thus to determine the position of the camera unit under the assumption that a light source is arranged in a ceiling or another predetermined location. One or more optical components 110 to 114 may also comprise an acceleration sensor for measuring the direction of the accelerations and the gravity of the camera unit 100 by means of the direction of three dimensions. When the camera unit 100 is in position or in a smooth movement, the direction of gravity may be determined and, based thereon, the position of the camera unit 100 may be concluded. When the position of the camera unit 100 has been found out relative to the direction of gravity, the position in which the image was taken with the camera unit 100 is known. The image may be rotated by means of this information to the desired position. Generally, the desire is to rotate an image for instance such that the vertical direction of the image points at an actual vertical direction determined based on gravity.

Based on the data obtained from one or more optical components 110 to 114, image production can be controlled at least partly also in other places than in the camera unit 100.

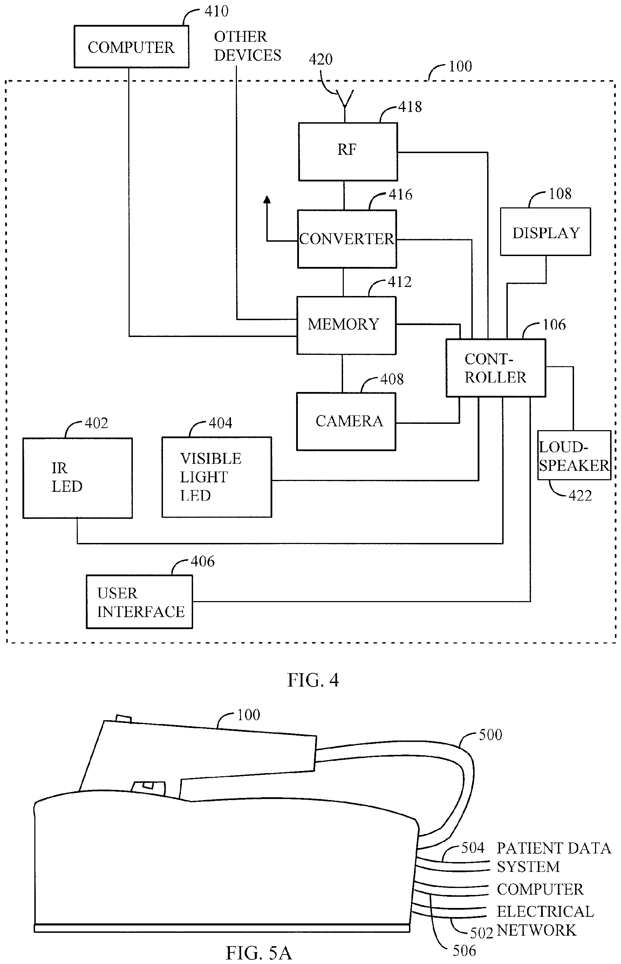

Let us now study block diagrams associated with the camera unit 100 and another feasible system by means of Figures 4 to 7. Figure 4 shows the block diagram of the camera unit 100. The camera unit 100 may comprise an infrared radiation source 402, a source of visible light 404, a user interface 406, a camera part 408, a controller 106 and a memory 412. The camera part 408 comprises a detector 104, among other things. The controller 106, which may comprise a processor and memory, may control the operation of the camera part 408. The controller 106 may receive the data associated with one or more optical components 110 to 114, and control image production for instance by adjusting the illumination, image brightness, image contrast, image colour saturation, colour, etc. The image can be transferred from the camera part 408 to the memory 412, from where the image can be transferred, controlled by the controller 106, to the display of the user interface 406 and/or elsewhere. Fixed images or video image taken of the object to be imaged may be stored in the memory 412, which may be of the flash type and repeatedly detachable and attachable, such as an SD memory card (Secure Digital). The memory 412 may also be located in a component 110 to 114.

The camera unit 100 may display real-time video image or a fixed image (e.g. the latest one taken) of the object to be imaged on the display 108. Image processing for facilitating a diagnosis may be performed based on the data associated with one or more optical components 110 to 114. Image processing, such as adjustment of contrast, adjustment of brightness, adjustment of colour saturation, adjustment of colouring or adjustment of illumination, for example, may be performed in connection with the imaging by means of the controller 106, for example. As a processing procedure, text or a scale can be added to an image taken or different images may be arranged superimposed. In addition, auditory or acoustic data may also be added to an image.

Each optical component 110 to 114 may comprise one or more microphones 136 for receiving acoustic data and for transferring to the camera unit 100 or for transmitting further via a radio transmitter 134, for example. The microphone 136 may be in a wired connection to the controller 106 when the optical component is connected to the camera unit 100. The camera unit 100 may also comprise a microphone 142, which is in connection to the controller 106. If more than one microphone 136, 142 is in use, one microphone may be intended to receive a patient's voice and another microphone may be intended to receive the voice of the examining person. One microphone, in turn, may be used to receive the voice of either the examining person or the patient or the voices of both the examining person and the patient.

Processing may also be performed in a docking station 550 or a base station 600 (Figures 5A, 5B, 6 and 7) either in real time or in connection with data transfer by means of the data associated with one or more optical components 110 to 114 comprised by the data structure 116 to 120.

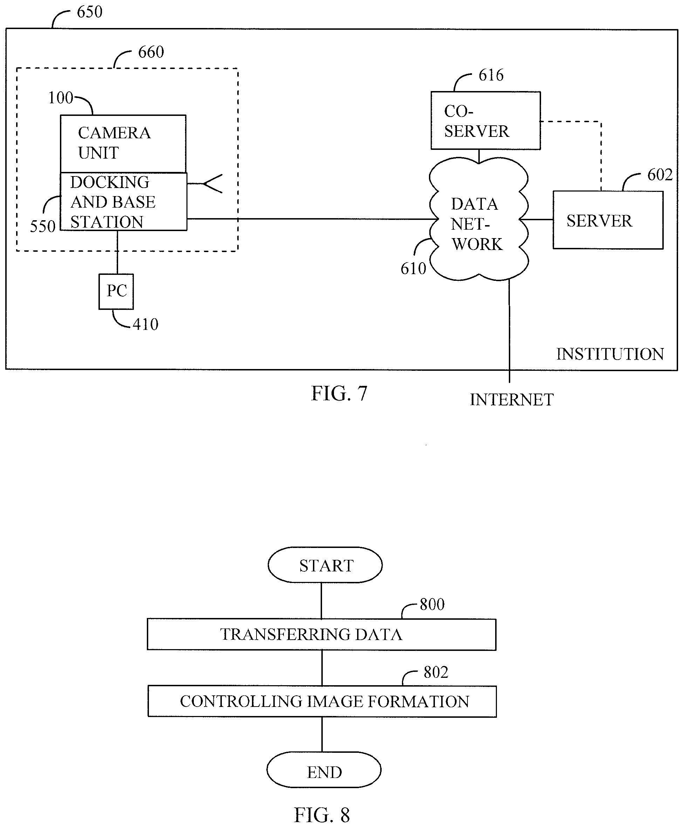

During data transfer from the camera part 408 towards the server 602 (Figures 6 and 7), when required, the data may be converted to conform to a suitable standard in a converter 416. The camera part 408 does not necessarily have a converter 416, but alternatively, it may be disposed in the docking station 550 or in the base station 600 (Figures 6 and 7). The converter 416 may convert the data format used by the camera part 408, which may be the XML format (Extensible Markup Language), for example, into a data format conforming to the DICOM protocol (Digital Imaging and Communications in Medicine), for example. The converted data may then be transferred to RF means 418, where the data is mixed up to a radio frequency. The radio frequency signal may be transmitted via the antenna 420 as electromagnetic radiation, which is received by the base station 600 (Figures 6 and 7).

The antenna 420 may be used to receive electromagnetic radiation including data and transmitted by the base station 600 (Figures 6 and 7). From the antenna 420, the signal propagates to the RF means 418, wherein the radio frequency signal is mixed down into baseband data. The converter 416 is able to convert the data format of the data received into another format, if need be. In this case, for example, the DICOM data format may be converted into the data format used by the camera unit 100, for example. The converted data may be transferred to the memory 412 and, when required, combined in the desired manner with an image taken by the camera part 408 in a manner controlled by the controller 106. From the memory 412, the data may be transferred to the display 108, for example, and to an optional loudspeaker 422.

If the camera unit 100 uses an SDIO card (Secure Digital Input Output), the radio link may be implemented directly with a WLAN connection or a Bluetooth® connection supported by the SDIO card. An SDIO card can also be used in the base station 600 and the docking station 550 for the radio link. The radio link may also be implemented in such a manner that an USB connector, to which an USB-WLAN adapter is fastened, is disposed within the camera unit 100, or the circuit board of the camera unit 100 is provided with a WLAN transceiver.

Each optical component 110 to 114 may comprise a separate communication component 134, such as a radio transmitter, a radio receiver or a radio transceiver. The communication component 134 may be in a wired connection to the controller 106 when the optical component is connected to the camera unit 100. The camera unit 100 may control the radio component 134 and use it for communication with the environment. The communication component 134 may also comprise a processor and memory, allowing it to process transferable data. This being so, the camera unit 100 is able to control the communication component 134 to acquire an IP address, for example, from the server 602. When the communication component 134 has established a connection via the base station 600 to the server 602, the camera unit 100 is able to perform data transfer with the server 602 in the DICOM format, for example.

The converter 416 is not necessarily required if the data format of the data to be transferred from the camera unit 100 to the base station 600 or even up to the server 602 remains the same.

The memory 412 is not necessarily required if the images taken with the camera unit 100 are transferred directly along the radio path to the base station 600. However, at least some kind of buffer memory is often required for securing the data transfer.

Data can be transferred from the analytical device also to the computer 410, which may include an image processing program. The computer 410 is able to process the image produced based on the data associated with one or more optical components 110 to 114 in the same manner as was described in the description of the camera unit 100. The computer 410 may receive data from the analytical device 660 wirelessly during the entire examination. When the physician is finished with the examination, the computer 410 is able to generate DICOM data, for example, from the data received thereby.

A still image or continuous video image, generated by the camera and processed by the image processing program, may be shown visually on the display of the computer 410. The user interface of the computer 410 may comprise a keyboard and a mouse for controlling the computer 410 and for inputting data. Image processing facilitating diagnosing, such as adjustment of illumination, adjustment of brightness, adjustment of contrast, adjustment of colouring and/or adjustment of colour saturation, may be performed directly in connection with the imaging, and the imaging can be monitored during the examination from the display of the computer 410.

Images may also be stored in the memory of the computer 410. As the memory may serve, a RAM memory (Random Access Memory) and a ROM memory (Read Only Memory), a hard disk, a CD disk (Compact disc) or corresponding memory means, known *per se.*

Figure 5A shows a camera unit 100 connected to a docking station 550. The analytical device 660 may comprise only a camera unit 100 or both a camera unit 100 and a docking station 550. The docking station 550 may be connected with a conductor 502 to a general electrical network, and the docking station 550 may use the electricity taken therefrom for its operation and convert it into a format required by the camera unit 100. Between the camera unit 100 and the docking station 550 is arranged a cable 500, along which the docking station 550 inputs, in the camera unit 100, the electrical power required thereby for loading a battery of the camera unit 100, for example. In addition, the docking station 550 may be designed such that the camera unit 100 may be placed in the docking station 550 steadily in position when the camera unit 100 is not used for examining an organ. The docking station 550 may also comprise a battery. The docking station 550 may be connected to a data network of a patient data system in a network with a conductor 504, or the docking station 550 may be in a wireless connection to the base station 600, which serves as an access point in the data transfer with the server 602. In addition, the docking station 550 may communicate with a PC 410, for example, over a cable 506.

Figure 5B shows a block diagram of a docking station. When the intention is to transfer data between the camera unit 100 and the docking station 550, the docking station 550 may comprise a signal processing unit 532, which comprises a processor and the required signal processing program, a memory 534, a converter 536, RF parts 538 and an antenna 540, which may correspond to the controller 106, the converter 416, the RF means 418 and the antenna 420 of the camera unit 100. In addition to the RF connection, the docking station 550 may be provided with another wireless connection 542 and/or a wired connection with a connector 544 before or after the converter 536. The wireless connection may operate for instance with a WLAN radio, a WIMAX radio (World Interoperability for Microwave Access), a Bluetooth radio or a WUSB radio (Wireless USB). The connector of the wired connection may be for instance a USB connector, a Firewire connector or a connector of a fixed network. In addition to or instead of being connected to a general electric network, the docking station 550 may comprise an operating loadable battery as the power source (not shown in Figure 5B).

The signal processing unit 532 may also be a controller and attend also to the control of the operation of the docking station 550 and image production based on the data associated with one or more optical components 110 to 114, in the same way as was described for the camera unit 100. However, the signal processing unit 532 is not necessarily required if the desired image production, modification and operation control are performed with the camera unit 100, the base station 600 or the server 602. The memory 534 is not necessarily either required.

However, the converter 536 of the docking station 550 may be required in case the camera unit 100 does not include a converter 416, but data format conversion is required. The RF means 538 of the docking station can be used to mix the data into a radio frequency signal that can be transmitted from the antenna 540 to the base station 600. Similarly, the antenna 540 of the docking station may receive radio frequency signals containing data from the base station 600. The signal received may be mixed with the RF parts 538 down into data and convert the data format thereof with the converter 536 when required. Furthermore, the data may be signalled to the camera unit 100 as data to be displayed to a user or as data for controlling the camera unit 100. The docking station 550 may also be in a fixed or wireless connection with the computer 410 and, over a network, to the server 602.

The docking station 550 is able to control the operation of the camera unit 100 and the data transfer between the camera unit 100 and the server 602 of the patient data system, provided the camera unit supports that the docking station controls of the operation thereof. The same docking station 550 may control the operation and data transfer of the different camera units 100 or other analytical devices in various manners, among other things for the reason that the different camera units 100 may be used for different analytical purposes.

The docking station 550 may control the image production of the camera unit 100 and the storage of the image in the memory of the camera unit 100, the docking station 550, the computer 410 or the server 602. Generally, the docking station 550 may control the storage of the data of the camera unit 100 in different memories.

The docking station 550 may control the image processing during the imaging. In this case, the image may be modified, analyzed, and the image may be stored. For example, the docking station 550 may convert the data received thereby from the camera unit into the DICOM format.

The docking station 550 may control the retrieval of patient data from the server 602 or the computer 410, and the storage of the data in the server 602 and the computer 410.

The docking station 550 may control the incorporation of patient data into an image produced by the camera unit 100. Generally, the docking station may control the incorporation of patient data into the data produced with the camera unit 100.

Let us now study some options for implementing the data transfer between the analytical device 660 and the server 602 of the patient data system by means of Figures 6 and 7. The analytical device 660 may be a whole comprised by the camera unit 100 and the docking station 550. The server block 602 of the patient data system may also comprise a coserver 616 and a centralized server, although they may also be physically separate. When data transfer is operating, the server 602 may also serve as a controller and thus control the camera unit 100. Image production may also be controlled by means of the data associated with one or more optical components 110 to 114 with the server 602 operating as a controller in the same way as was described for the camera unit 100.

In Figure 6, the base station 600 and the docking station 550 of a hospital (or another institution) are shown as separate. The camera unit 100 may include means for wireless connection with the docking station 550 or the camera unit 100 and the docking unit 550 may perform data transfer only over a wired connection or a direct connection. Alternatively or in addition, the camera unit 100 may comprise means for being in a wireless connection directly to the base station 600.

When the camera unit 100 is started, the camera unit 100 may automatically establish a connection to the base station 600, or the user controls the camera unit 100 manually to establish a connection to the base station 600. Alternatively or in addition, the camera unit 100 may establish a connection to the docking station 550, which may optionally perform necessary processing of the image. The docking station 550, in turn, may establish a connection or already have a connection with the base station 600, whereby a connection is established between the camera unit 100 and the base station 600. The base station 600, in turn, may have a wired connection, over a data network 610, for example, to the server 602, which is an internal server of a patient data system of a hospital or another similar institution 650. In this case, the base station 600 may serve as an access point between the wireless and the wired systems. The camera unit 100, the docking station 550 and the base station 600 may transmit and receive radio-frequency electromagnetic radiation. This may be implemented by using RF parts (Radio Frequency), for example, the basis of the operation thereof being WLAN (Wireless Local Area Network), Bluetooth® and/or a mobile telephony technique, such as GSM (Global System for Mobile Communication) or WCDMA (Wideband Code Division Multiple Access), for example. When the Bluetooth® solution is used, the coverage is dozens of metres, with the WLAN solution the coverage is hundreds of metres and the coverage of a mobile telephony technique may be up to dozens of kilometres.

The base station 600 and the docking station 550 may also be combined into one device, as is shown in Figure 7, whereby it may be imagined that the docking station 550 also includes the base station functions. In this case, a separate base station 600 is not necessarily required. Thus, the analytical device 660 may be in a wired connection to the data network 610 and serve as a base station for other devices being in connection or seeking to establish a connection with the server 602.

When the camera unit 100 is connected to the docking station 550 with a USB connector (Universal Serial Bus), for example, data can be transferred from the camera unit 100 to the docking station 550, from where the data can be further transmitted in real time or after a desired delay to the base station 600 and further to the server 602. When data originating from one or more optical components 110 to 114 and indicating that the image is intended only for specialist physicians according to the imaging object, for example, has been attached to the image, only said specialist physicians have access to the image. Data can be transferred from the server 602 to the base station 600, which transmits the data to the docking station 550 and further to the camera unit 100. The transfer from the server 602 to the camera unit 100 is successful if the distribution of the data to be transferred has not been restricted or if the user of the camera unit 100 is authorized to receive the data. In addition, the docking station 550 may be in connection to the computer 410, to which the connection may be implemented by using a USB connection or a LAN network (Local Area Network), for example.

Instead of a wireless connection, the docking station 550 may also be in a wired connection to the data network 610. Furthermore, the camera unit 100 may be in connection to a computer 410, such as a PC (Personal Computer). The connection to the computer 410 may be implemented by using a USB connection, for example.

When the analytical device 660 is connected to the data network 608, 610 or to the patient data system, the camera unit 100 is able to transmit its identifier to the coserver 616. The identifier of the docking station 550 may be included. The identifier may be transmitted to the coserver 616 secured over a secure connection 608, 610, 612, for example. The identifier may be an identification of the device, which may be in a textual format, for example, and which may comprise the name and model of the device, for example. The coserver 616 may receive the identifier defining the camera unit 100 and a possible docking station 550 over the secure connection 608, 610, 612.

The coserver 616 has access to installation data associated with at least one accepted identifier of the device. The coserver 616 may be a server located in a hospital, in which coserver the data on the device acquired required in connection with the acquirement of each device is stored and updated. The data can be input manually and retrieved automatically from the manufacturer's server, for example. The coserver 616 may also be a server maintained by the manufacturer, from which data associated with each device can be retrieved directly via the Internet, for example.

The coserver 616 is able to transmit the installation data associated with the identifier of the analytical device 660 to the server 602 of the patient data system. The transmission may be performed over the secure connection 608, 610, 612, 614.

The secure connection 608, 610, 612, 614 may be an internal and protected data network of the hospital or a data network extending at least partly outside the hospital, in which network the data to be transferred can be secured by an encryption program. One such form of connection is the SSH (Secure SHell), which may be used in logging in the server.

The identifier defining the analytical device 660 may be installed automatically based on the installation data in the server 602 of the patient data system for data transfer. Once the identifier is installed, data transfer can be initiated between the analytical device 660 and the server 602 of the patient data system. Similarly, the identifier of any other device may be installed in the same way in the server 602 and data transfer initiated. This enables the authentication of either the camera unit 100, the docking station 550 or the entire analytical device 660. If the authentication of the analytical device 660 fails, the server 602 may notify of the problem. The quality of the problem may also be notified. For example, it may be a question of a wrong kind of formation of the different fields in the data format or a data format unsuitable for the server 602. The docking station 550 or the base station 600 may process the data to a format suitable for the server 602 and thus the formation of the fields and the data format, for example, may be corrected. Alternatively, the docking station 550 may control the camera unit 100 to process the data into a format suitable for the server 602.

Figure 8 shows a flow diagram of the method. In step 800, data associated with an optical component 110 to 114 is transferred from a data structure 116 to 120 to the camera unit 100 when at least one optical component 110 to 114 is connected to the camera unit 100. In step 802, the formation of an image produced by the camera unit 100 of an organ is controlled based on data associated with one or more optical components 110 to 114.

Without data transferred from the optical connection in connection with an image event, or in addition thereto, the user is able to input an imaging object in the camera unit, provided the camera unit operates in a different manner in relation to different imaging objects. The user is also able to set luminosity, colour or other rules, control the values associated with different illumination to be suitable via menus of the camera unit, and estimate the accuracy of the image either him/herself from the display of the device or in a PC apparatus after image transfer.

Although the invention is described above with reference to the examples in accordance with the accompanying drawings, it will be appreciated that the invention is not to be so limited, but it may be modified in a variety of ways within the scope of the appended claims.

## Claims

1. A method of processing an image with an analytical device for producing images of organs in an electronic format, wherein the analytical device comprises:

   a camera unit (100), comprising a camera objective (102) and a detector (104);
   and at least a first and a second optical component (110 to 114) fastenable to the camera unit (100);
   wherein the camera unit (100), without any of the optical components (110 to 114) fastened, is adapted to capture images of skin being the organ;
   wherein the camera unit (100) is adapted to capture images of a nose, ear or mouth cavity as the organ, if the second optical component (114) is fastened to said camera unit;
   wherein the camera unit (100) is adapted to capture images of the tympanic membrane or of the external acoustic meatus as the organ, if the first (110) and second optical component (114) are fastened to said camera unit;
   wherein the first and the second optical components (110 to 114) include respective data structures (116 to 120) including data associated with the respective optical component (110 to 114) and the organ to be imaged; the method comprising at least one of: using the camera unit (100) without any of the optical components (110, 114) fastened for capturing an image of skin;
   using the camera unit (100) to capture an image of a nose, ear or mouth cavity when the second optical component (114) is fastened to said camera unit (100);
   and using the camera unit (100) to capture an image of the tympanic membrane or of the external acoustic meatus when the first and the second optical components (110, 114) are fastened to said camera unit (100); the method further comprising:

      transferring (800), whenever a respective one of the optical components (110 to 114) is fastened to the camera unit (100), the data from the corresponding data structure (116 to 120) to the camera unit (100); and controlling (802) the image production by the camera unit (100) based on the transferred data.

2. A method as claimed in claim 1, **characterized by** transferring data associated with the first or the second optical component (110 to 114) connected to the camera unit (100) from the corresponding data structure (116 to 120) to a controller (106) of the camera unit (100), and controlling the image production with the controller (106) based on the transferred data.

3. An analytical device for producing an image, the analytical device (660) being adapted to produce images in an electronic format of organs with a detector (104), wherein the analytical device (660) comprises: a camera unit (100) comprising: a camera objective, and a detector (104); and at least a first and a second optical component (110 to 114) fastenable to the camera unit (100); the camera unit (100) without any of the optical components fastened being configured to capture an image of skin; the camera unit (100) being configured to capture an image of a nose, ear or mouth cavity when the second optical component is fastened to said camera unit (100); the camera unit (100) being configured to capture an image of the tympanic membrane or of the external acoustic meatus when the first and the second optical components (110, 114) are fastened to said camera unit (100); each optical component (110 to 114) including a corresponding data structure (116 to 120) including data associated with the respective optical component (110 to 114) and the organ to be imaged; the data structure (116 to 120) of each optical component (110 to 114) being adapted to transfer the data from the data structure (116 to 120) to the camera unit (100) when the respective optical component (110 to 114) is fastened to the camera unit (100); and the camera unit (100) being adapted to control the production of the image based on the transferred data.

4. An analytical device as claimed in claim 3, wherein the camera unit further comprises a controller (106), **characterized in that** the data structures (116 to 120) are memory circuits wherein said memory circuits and the controller (106) are adapted to communicate with each other for transferring the data associated with the optical component (110 to 114) to the controller (106), and the controller (106) is adapted to control image production based on the transferred data.

5. An analytical device as claimed in claim 3, **charac-**

**terized in that** the data structures (116 to 120) are adapted to control the brightness of the image based on the data associated with the respective optical component (110 to 114).

6. An analytical device as claimed in claim 3, **characterized in that** the data structures (116 to 120) are adapted to control the contrast of the image based on the data associated with the respective optical component (110 to 114).

7. An analytical device as claimed in claim 3, **characterized in that** the data structures (116 to 120) are adapted to control the colour shades of the image based on the data associated with the respective optical component (110 to 114).

8. An analytical device as claimed in claim 3, **characterized in that** the data structures (116 to 120) are adapted to control the colouring of the image on at least part of the image area based on the data associated with the respective optical component (110 to 114).

9. An analytical device as claimed in claim 4, **characterized in that** the data structures (116 to 120) are adapted to control the controller (106) of the camera unit (100) to crop part of the detection area of the detector (104) based on the data associated with the optical component (110 to 114).

10. An analytical device as claimed in claim 3, **characterized in that** the data structures (116 to 120) are adapted to control image production based on the data associated with the respective optical component (110 to 114) by producing the image with predetermined exposure values.

11. An analytical device as claimed in claim 3, **characterized in that** the camera unit (100) comprises at least one radiation source (124, 126) adapted to illuminate organs to be imaged; and the data structures (116 to 120) are adapted to control image production based on the data associated with the respective optical component (110 to 114) by adjusting the at least one radiation source (124, 126) of the camera unit (100).

12. An analytical device as claimed in claim 3, **characterized in that** the data structures (116 to 120) are adapted to control image production based on the data associated with the respective optical component (110 to 114) by adjusting the illumination of an examination room (652).

13. An analytical device as claimed in claim 3, **characterized in that** the camera unit (100) is adapted to attach data on one or more optical components (110 to 114) used to the image.

14. An analytical system for producing an image, the analytical system comprising an analytical device (660) of claim 3 for producing an image of an organ in an electronic format, **characterized in that** the analytical system comprises a server (602); wherein the server (602) and the analytical device (660) are adapted to be in connection with each other.

**Patentansprüche**

1. Bildverarbeitungsverfahren mit einer analytischen Vorrichtung zur Bilderzeugung von Organen in einem elektronischen Format, wobei die analytische Vorrichtung umfasst:

    eine Kameraeinheit (100), umfassend ein Kameraobjektiv (102) und einen Detektor (104); und mindestens eine erste und eine zweite optische Komponente (110 bis 114) befestigbar an der Kameraeinheit (100) ;
wobei die Kameraeinheit (100), ohne eine der optischen Komponenten (110 bis 114) befestigt, angepasst ist zum Aufnehmen von Bildern der Haut, die das Organ ist;
wobei die Kameraeinheit (100) angepasst ist zum Aufnehmen von Bildern von einer Nase, einem Ohr oder einer Mundhöhle als das Organ, wenn die zweite optische Komponente (114) an der Kameraeinheit befestigt ist;
wobei die Kameraeinheit (100) zum Aufnehmen von Bildern des Trommelfells oder des äußeren Gehörgangs als das Organ angepasst ist, wenn die erste (110) und zweite optische Komponente (114) an der Kameraeinheit befestigt sind;
wobei die ersten und die zweiten optischen Komponenten (110 bis 114) jeweilige Datenstrukturen (116 bis 120) enthalten, die Daten zugehörig zu der jeweiligen optischen Komponente (110 bis 114) und dem abzubildenden Organ enthalten; wobei das Verfahren mindestens eines umfasst von:

    Anwenden der Kameraeinheit (100) ohne eine der optischen Komponenten (110, 114) befestigt zum Aufnehmen eines Bildes der Haut;
Anwenden der Kameraeinheit (100) zum Aufnehmen eines Bildes von einer Nase, einem Ohr oder einer Mundhöhle, wenn die zweite optische Komponente (114) an der Kameraeinheit (100) befestigt ist; und
Anwenden der Kameraeinheit (100) zum Aufnehmen eines Bildes des Trommelfells oder des äußeren Gehörgangs, wenn die ersten und die zweiten optischen Komponenten (110, 114) an der Kameraeinheit (100) befestigt sind;

wobei das Verfahren weiterhin umfasst:

    Übertragen (800), jedes Mal, wenn eine jeweilige der optischen Komponenten (110 bis 114) an der Kameraeinheit (100) befestigt ist, der Daten von der entsprechenden Datenstruktur (116 bis 120) zu der Kameraeinheit (100) ; und
Steuern (802) der Bilderzeugung durch die Kameraeinheit (100), basierend auf den übertragenen Daten.

2. Verfahren nach Anspruch 1, **gekennzeichnet durch** Übertragen der zu der ersten oder der zweiten, mit der Kameraeinheit (100) verbundenen, optischen Komponente (110 bis 114) zugehörigen Daten, von der entsprechenden Datenstruktur (116 bis 120) zu einem Controller (106) der Kameraeinheit (100), und Steuern der Bilderzeugung mit dem Controller (106), basierend auf den übertragenen Daten.

3. Analytische Vorrichtung zur Erzeugung eines Bildes, wobei die analytische Vorrichtung (660) zum Erzeugen von Bildern in einem elektronischen Format von Organen mit einem Detektor (104) angepasst ist, wobei die analytische Vorrichtung (660) umfasst:

    eine Kameraeinheit (100), umfassend:

        ein Kameraobjektiv und einen Detektor (104); und mindestens eine erste und eine zweite optische Komponente (110 bis 114) befestigbar an der Kameraeinheit (100);
wobei die Kameraeinheit (100), ohne dass eine der optischen Komponenten befestigt ist, zum Aufnehmen eines Bildes der Haut ausgelegt ist;
wobei die Kameraeinheit (100) zum Aufnehmen eines Bildes von einer Nase, einem Ohr oder einer Mundhöhle ausgelegt ist, wenn die zweite optische Komponente an der Kameraeinheit (100) befestigt ist;
wobei die Kameraeinheit (100) zum Aufnehmen eines Bildes des Trommelfells oder des äußeren Gehörgangs ausgelegt ist, wenn die ersten und die zweiten optischen Komponenten (110, 114) an der Kameraeinheit (100) befestigt sind;
wobei jede optische Komponente (110 bis 114) eine entsprechende Datenstruktur (116 bis 120) enthält, die Daten zugehörig zu der jeweiligen optischen Komponente (110 bis 114) und dem abzubildenden Organ enthält;
wobei die Datenstruktur (116 bis 120) von jeder optischen Komponente (110 bis 114) zum Übertragen der Daten von der Datenstruktur (116 bis 120) zu der Kameraeinheit (100) angepasst ist, wenn die jeweilige optische Komponente (110 bis 114) an der Kameraeinheit (100) befestigt ist; und wobei die Kameraeinheit (100) zum Steuern der Erzeugung des Bildes, basierend auf den übertragenen Daten, angepasst ist.

4. Analytische Vorrichtung nach Anspruch 3, wobei die Kameraeinheit weiterhin einen Controller (106) umfasst, **dadurch gekennzeichnet, dass** die Datenstrukturen (116 bis 120) Speicherkreise sind, wobei die Speicherkreise und der Controller (106) angepasst sind zum Kommunizieren miteinander zum Übertragen der Daten zugeordnet zu der optischen Komponente (110 bis 114) an den Controller (106), und der Controller (106) zum Steuern der Bilderzeugung, basierend auf den übertragenen Daten, angepasst ist.

5. Analytische Vorrichtung nach Anspruch 3, **dadurch gekennzeichnet, dass** die Datenstrukturen (116 bis 120) angepasst sind zum Steuern der Helligkeit des Bildes, basierend auf den Daten zugehörig zu der jeweiligen optischen Komponente (110 bis 114).

6. Analytische Vorrichtung nach Anspruch 3, **dadurch gekennzeichnet, dass** die Datenstrukturen (116 bis 120) angepasst sind zum Steuern des Kontrasts des Bildes, basierend auf den Daten zugehörig zu der jeweiligen optischen Komponente (110 bis 114).

7. Analytische Vorrichtung nach Anspruch 3, **dadurch gekennzeichnet, dass** die Datenstrukturen (116 bis 120) angepasst sind zum Steuern der Farbschattierungen des Bildes, basierend auf den Daten zugehörig zu der jeweiligen optischen Komponente (110 bis 114).

8. Analytische Vorrichtung nach Anspruch 3, **dadurch gekennzeichnet, dass** die Datenstrukturen (116 bis 120) angepasst sind zum Steuern der Färbung des Bildes auf mindestens einem Teil der Bildfläche, basierend auf den Daten zugehörig zu der jeweiligen optischen Komponente (110 bis 114).

9. Analytische Vorrichtung nach Anspruch 4, **dadurch gekennzeichnet, dass** die Datenstrukturen (116 bis 120) angepasst sind zum Steuern des Controllers (106) der Kameraeinheit (100) zum Beschneiden eines Teils der Detektionsfläche des Detektors (104), basierend auf den Daten zugehörig zu der optischen Komponente (110 bis 114).

10. Analytische Vorrichtung nach Anspruch 3, **dadurch gekennzeichnet, dass** die Datenstrukturen (116 bis 120) angepasst sind zum Steuern der Bilderzeugung, basierend auf den Daten zugehörig zu der jeweiligen optischen Komponente (110 bis 114) durch Erzeugen des Bildes mit vorbestimmten Belichtungswerten.

11. Analytische Vorrichtung nach Anspruch 3, **dadurch gekennzeichnet, dass** die Kameraeinheit (100) mindestens eine Strahlungsquelle (124, 126) umfasst, angepasst zum Beleuchten der abzubildenden Organe; und die Datenstrukturen (116 bis 120) angepasst sind zum Steuern der Bilderzeugung, basierend auf den Daten zugehörig zu der jeweiligen optischen Komponente (110 bis 114) durch Einstellen der mindestens einen Strahlungsquelle (124, 126) der Kameraeinheit (100).

12. Analytische Vorrichtung nach Anspruch 3, **dadurch gekennzeichnet, dass** die Datenstrukturen (116 bis 120) angepasst sind zum Steuern der Bilderzeugung, basierend auf den Daten zugehörig zu der jeweiligen optischen Komponente (110 bis 114) durch Einstellen der Beleuchtung eines Untersuchungszimmers (652).

13. Analytische Vorrichtung nach Anspruch 3, **dadurch gekennzeichnet, dass** die Kameraeinheit (100) angepasst ist zum Einsetzen der Daten auf einer oder mehreren optischen Komponenten (110 bis 114), die für das Bild verwendet werden.

14. Analytisches System zum Erzeugen eines Bildes, wobei das analytische System eine analytische Vorrichtung (660) nach Anspruch 3 zum Erzeugen eines Bildes von einem Organ in einem elektronischen Format umfasst, **dadurch gekennzeichnet, dass** das analytische System einen Server (602) umfasst; wobei der Server (602) und die analytische Vorrichtung (660) angepasst sind, um miteinander in Verbindung zu sein.

**Revendications**

1. Procédé de traitement d'une image avec un dispositif analytique pour produire des images d'organes dans un format électronique, dans lequel le dispositif analytique comprend :

une unité de caméra (100), comprenant un objectif de caméra (102) et un détecteur (104) ; et au moins un premier et un deuxième composants optiques (110 à 114) pouvant être fixé à l'unité de caméra (100) ; dans lequel l'unité de caméra (100), sans aucun des composants optiques (110 à 114) fixés, est adaptée pour capturer des images de la peau étant l'organe ;

dans lequel l'unité de caméra (100) est adaptée pour capturer des images d'une cavité nasale, auditive ou buccale en tant qu'organe, si le deuxième composant optique (114) est fixé à ladite unité de caméra ;

dans lequel l'unité de caméra (100) est adaptée pour capturer des images de la membrane tympanique ou du méat acoustique externe comme l'organe, si le premier (110) et le deuxième (114) composants optiques sont fixés à ladite unité de caméra ;

dans lequel le premier et le deuxième composants optiques (110 à 114) incluent des structures de données respectives (116 à 120) incluant des données associées au composant optique respectif (110 à 114) et à l'organe dont on souhaite une image ; le procédé comprenant au moins un de :

utiliser l'unité de caméra (100) sans aucun des composants optiques (110 à 114) fixés pour capturer une image de peau ;

utiliser l'unité de caméra (100) pour capturer une image d'une cavité nasale, auditive ou buccale lorsque le deuxième composant optique (114) est fixé à ladite unité de caméra (100) ;

et utiliser l'unité de caméra (100) pour capturer une image de la membrane tympanique ou du méat acoustique externe lorsque le premier et le deuxième composants optiques (110 à 114) sont fixés à ladite unité de caméra (100) ;

le procédé comprenant en outre :

transférer (800), chaque fois que l'un des composants optiques respectifs (110 à 114) est fixé à l'unité de caméra (100), des données des structures de données correspondantes (116 à 120) à l'unité de caméra (100) ; et

contrôler (802) la production d'image par l'unité de caméra (100) sur la base des données transférées.

2. Procédé selon la revendication 1, **caractérisé par** le transfert des données associées au premier et au deuxième composants optiques (110 à 114) connectés à l'unité de caméra (100) à partir des structures de données correspondantes (116 à 120) à un contrôleur (106) de l'unité de caméra (100), et le contrôle de la production d'image avec le contrôleur (106) sur la base des données transférées.

3. Dispositif analytique pour la production d'une image, le dispositif analytique (660) étant adapté pour produire des images dans un format électronique d'organes avec un détecteur (104), dans lequel le dispositif analytique (660) comprend : une unité de caméra (100) comprenant : un objectif de caméra , et un détecteur (104) ; et au moins un premier et un deuxième composants optiques (110 à 114) pouvant être fixés à l'unité de caméra (100) ; l'unité de caméra (100) sans aucun des composants optiques fixés étant configurée pour capturer une image de peau ; l'unité de caméra (100) étant configurée pour capturer une image d'une cavité nasale, auditive ou buccale lorsque le deuxième composant optique est fixé à ladite unité de caméra (100) ; l'unité de caméra (100) étant configurée pour capturer une image de la membrane tympanique ou du méat acoustique externe lorsque le premier et le deuxième composants optiques (110 à 114) sont fixés à ladite unité de caméra (100) :

chaque composant optique (110 à 114) incluant une structure de données correspondante (116 à 120) incluant des données associées au composant optique respectif (110 à 114) et à l'organe dont on souhaite une image ;

la structure de données (116 à 120) de chaque composant optique (110 à 114) étant adaptée pour transférer les données de la structure de données (116 à 120) à l'unité de caméra (100) lorsque le composant optique respectif (110 à 114) est fixé à l'unité de caméra (100) et ;

l'unité de caméra (100) étant adaptée pour contrôler la production de l'image sur la base des données transférées.

4. Dispositif analytique selon la revendication 3, dans lequel l'unité de caméra comprend en outre un contrôleur (106), **caractérisé en ce que** les structures de données (116 à 120) sont des circuits de mémoire dans lequel lesdits circuits de mémoire et le contrôleur (106) sont adaptés pour communiquer les uns avec les autres pour transférer les données associées au composant optique (110 à 114) au contrôleur (106), et le contrôleur (106) est adapté pour contrôler la production d'image sur la base des données transférées.

5. Dispositif analytique selon la revendication 3, **caractérisé en ce que** les structures de données (116 à 120) sont adaptées pour contrôler la luminosité de l'image sur la base des données associées au composant optique respectif (110 à 114).

6. Dispositif analytique selon la revendication 3, caractérisé en que les structures de données (116 à 120) sont adaptées pour contrôler le contraste de l'image sur la base des données associées au composant optique respectif (110 à 114).

7. Dispositif analytique selon la revendication 3, **carac-**

**térisé en ce que** les structures de données (116 à 120) sont adaptées pour contrôler les nuances de couleurs de l'image sur la base des données associées au composant optique respectif (110 à 114).

8. Dispositif analytique selon la revendication 3, **caractérisé en ce que** les structures de données (116 à 120) sont adaptées pour contrôler la coloration de l'image sur au moins une partie de la zone d'image sur la base des données associées au composant optique respectif (110 à 114).

9. Dispositif analytique selon la revendication 4, **caractérisé en ce que** les structures de données (116 à 120) sont adaptés pour contrôler le contrôleur (106) de l'unité de caméra (100) pour rogner une partie de la zone de détection du détecteur (104) sur la base des données associées au composant optique respectif (110 à 114).

10. Dispositif analytique selon la revendication 3, **caractérisé en ce que** les structures de données (116 à 120) sont adaptées pour contrôler la production d'image sur la base des données associées au composant optique respectif (110 à 114) en produisant l'image avec des valeurs d'exposition prédéterminées.

11. Dispositif analytique selon la revendication 3, **caractérisé en ce que** l'unité de caméra (100) comprend au moins une source de rayonnement (124, 126) adaptée pour illuminer les organes dont on souhaite une image ; et
les structures de données (116 à 120) sont adaptées pour contrôler la production d'image sur la base des données associées au composant optique respectif (110 à 114) en ajustant l'au moins une source de radiation (124, 126) de l'unité de caméra (100).

12. Dispositif analytique selon la revendication 3, **caractérisé en ce que** les structures de données (116 à 120) sont adaptées pour contrôler la production d'image sur la base des données associées au composant optique respectif (110 à 114) en ajustant l'illumination d'une pièce d'examen (652).

13. Dispositif analytique selon la revendication 3, **caractérisé en ce que** l'unité de caméra (100) est adaptée pour attacher des données sur un ou plusieurs composants optiques (110 à 114) utilisés à l'image.

14. Système analytique pour la production d'une image, le système analytique comprenant un dispositif analytique (660) selon la revendication 3 pour la production d'une image d'un organe dans un format électronique, **caractérisé en ce que** le système analytique comprend un serveur (602) ;
dans lequel

le serveur (602) et le dispositif analytique (660) sont adaptés pour être en connexion l'un avec l'autre.

FIG. 1

FIG. 2A

FIG. 2B

FIG. 3

FIG. 4

FIG. 5A

FIG. 5B

FIG. 6

FIG. 7

INSTITUTION

INTERNET

FIG. 8

START

TRANSFERRING DATA

CONTROLLING IMAGE FORMATION

END

## REFERENCES CITED IN THE DESCRIPTION

**Patent documents cited in the description**

- US 20070030345 A
- US 6092722 A
- WO 9942030 A
- FI 107120
- FI 200212233